(19) Europäisches Patentamt
European Patent Office
Office européen des brevets

(11) EP 2 425 859 A1

(12) EUROPEAN PATENT APPLICATION

(43) Date of publication:
**07.03.2012 Bulletin 2012/10**

(21) Application number: **10172244.5**

(22) Date of filing: **08.08.2010**

(51) Int Cl.:
***A61K 47/10*** (2006.01)
***A61K 9/20*** (2006.01)
***A61K 9/16*** (2006.01)
***A61K 9/14*** (2006.01)
***A61K 9/50*** (2006.01)
***A61K 31/4178*** (2006.01)
***A61K 45/06*** (2006.01)

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Abdi Ibrahim Ilac Sanayi ve Ticaret Anonim Sirketi**
**Istanbul (TR)**

(72) Inventors:
- **Farshi, Farhad**
  **34555, Istanbul (TR)**
- **Avci, Recep**
  **34555, ISTANBUL (TR)**
- **Yildirim, Ersin**
  **34555, Istanbul (TR)**
- **Soylemez, Serdar**
  **34555, Istanbul (TR)**

(54) **Olmesartan formulations**

(57) The present invention provides pharmaceutical compositions comprising olmesartan or pharmaceutically acceptable salts and esters thereof or combination with active pharmaceutical ingredient or ingredients such as amlodipine or pharmaceutically acceptable salts and suitable additives for an improved dissolution profiles.

EP 2 425 859 A1

## Description

## Technical Field

The present invention provides pharmaceutical compositions comprising olmesartan or pharmaceutically acceptable salts and esters thereof or combination with an active pharmaceutical ingredient or ingredients such as amlodipine or pharmaceutically acceptable salts and suitable additives for an improved dissolution profiles.

## Background Art

Angiotensin II is a very potent chemical that causes muscles surrounding blood vessels to contract, thereby narrowing blood vessels. This narrowing increases the pressure within the vessels and can cause hypertension. Angiotensin II receptor blockers (ARBs) are medications that block the action of angiotensin II by preventing angiotensin II from binding to angiotensin II receptors on blood vessels. As a result, blood vessels enlarge (dilate) and blood pressure is reduced. Reduced blood pressure makes it easier for the heart to pump blood and can improve heart failure.
Drugs in this class include candesartan, eprosartan, losartan, olmesartan, telmisartan ,valsartan and pratosartan. ARBs are used alone or in combination with other classes of antihypertensive agents that include thiazide diuretics, blockers, calcium channel blockers, rennin inhibitor, and ACE inhibitors, both for the treatment of hypertension and congestive heart failure.
Olmesartan which is an angiotensin 11 receptor antagonist is pharmaceutically used as an antihypertensive for the treatment and prophylaxis of hypertension.
Its chemical name is 2,3-dihydroxy-2-butenyl 4-(I-hydroxy-I-methylethyl)-2-propyl-I-[j!7-(o-IH-tetrazol-5-ylphenyl)benzyl]imidazole-5-carboxylate, cyclic 2,3- carbonate or (5-methyl-2-oxo~1,3-dioxolen-4-yl)rnethyl 4-(I -hydroxy- I-methylethyl)-2- propyl- 1 - {4-[2-(tetrazol-5-yl)phenyl]phenyl}methylimidazole-5-carboxylate having the following structure:
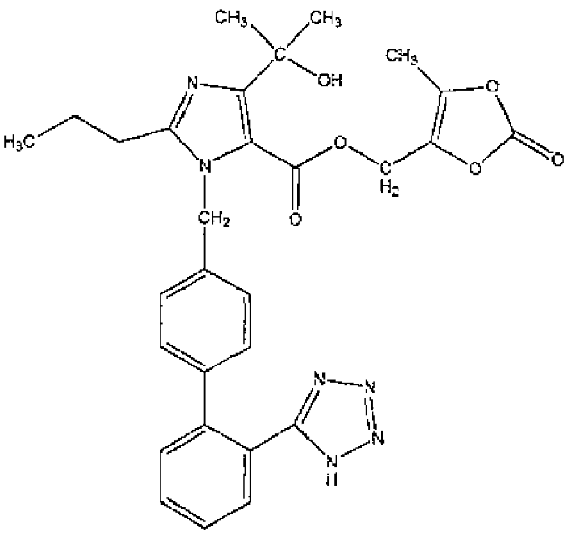

Olmesartan medoxomil was developed to achieve better oral bioavailability.
Olmesartan medoxomil is an ester prodrug of olmesartan which after ingestion liberates the only active metabolite. Olmesartan medoxomil is marketed by Sankyo under the trade name of Olmetec® or Benicar®. It is available as oral tablets in strengths of 5 mg, 10 mg, 20 mg and 40 mg.
Olmesartan medoxomil is practically insoluble in water and in the pH range of 3 to 9, sparingly soluble in strong acid (except insoluble in 17 hydrochloric acid), and soluble in strong base.
Amlodipine is a calcium channel blocker developed for the treatment of hypertension and other medical indications. Its chemical name is 3-ethyl-5-methyl-(±)-2-[(2-aminoethoxy)methyl]-4-(2-chlorophenyl)-I,4-dihydro-6-methylpyridine-3,5-dicarboxylate, having the following structure:

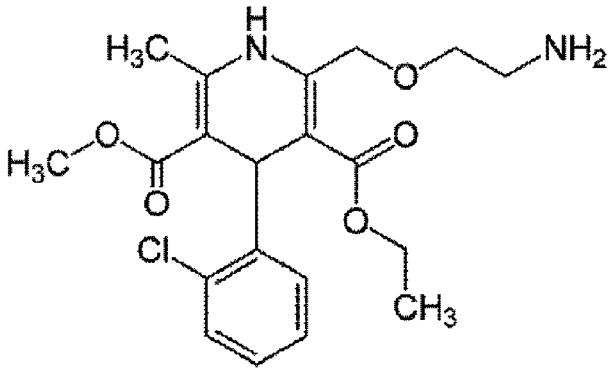

Certain solid pharmaceutical composition comprising olmesartan or pharmaceutically acceptable salts and esters thereof and process for their preparation are known.

Olmesartan was firstly disclosed by EP 0503785 B (SANKYO COMPANY LIMITED) 16.09.1992.

Amlodipine was firstly disclosed by EP 244944 B (PFIZER LIMITED) 11.11.1987.

Olmesartan medoxomil has a low bioavailability (BA), approximately 26% in humans, due to its low water solubility and efflux by drug resistance pumps in the gastrointestinal tract.

The production of drug products containing olmesartan requires formulation techniques which enhance the dissolution property of olmesartan.

Published WO 2008/149338 A (DEXCEL LTD.) 11.12.2008 discloses a novel method of preparation of a formulation comprising an angiotensin II receptor antagonist and in particular, to a method using melt granulation of solid components. In the pharmaceutical industry, the melt granulation technique has been used for improving the dissolution rate and bioavailability of a drug by forming a solid dispersion or solid solution.

Published EP 2033643 A (DAIICHI SANKYO LIMITED) 03.01.2008 discloses a production method olmesartan medoxomil containing drug product having an improved dissolution property.

Published WO 2007/001066 A (SANKYO COMPANY LIMITED) 04.01.2007 discloses a pharmaceutical preparation comprising an angiotensin II receptor antagonist a calcium channel blocker. A pharmaceutical preparation with improved dissolution properties is obtained by incorporation of at least one substance selected from a hydrophilic polymer, an acidic substance and a fluiziding agent.

## Summary of invention

It is an object of the present invention to provide a process for a solid pharmaceutical composition comprising olmesartan or pharmaceutically acceptable salts and esters thereof and an active pharmaceutical ingredient such as amlodipine or pharmaceutically acceptable salts combination wherein the solubility and bioavailability of olmesartan is improved with a mechanical process of mixing and sieving of active ingredient with poloxamer from an milling equipment of screen preferably 315 microns.

Another aspect of the present invention is that poloxamer is used as a solubility enhancer agent in a spesific amount of 0.1-15 %( w/w) of the total pharmaceutical composition.

This invention also embraces olmesartan or pharmaceutically acceptable salts and esters thereof and at least one pharmaceutically acceptable excipient.

### Technical Problem

In drug development, therapeutic effectiveness of a drug depends upon the bioavailability and ultimately upon the solubility of drug molecules. Bioavailability is a pharmacokinetic parameter defined by the amount of a drug absorbed based on the amount administered, which is used to determine the effectiveness of an administered pharmaceutical active ingredient or a formulation comprising same. The characteristics of a pharmaceutical active ingredient, e.g., water-solubility, crystal forms and particle size of the active ingredient can affect the bioavailability of the active ingredient or a composition comprising same. Low water solubility and low bioavailability are frequent problems which are directly related to the drug molecule and need to be solved. For example, the sparingly water-soluble drug itself is not absorbed in the gastrointestinal tract, leading to poor bioavailability.

Various techniques are available to improve the solubility of poorly soluble drugs. Chemical and physical properties of active ingredient can be modified.

Besides active ingredient's physical and chemical modifications, different formulation techniques which enhance the dissolution property of the active ingredient are generally used.

Although use of solvent recrystallization, inclusion in cyclodextrin- drug complexes and novel technologies such as super critical fluid processing, micronization via nanoparticles are widespread possess significant limitations by means of productions.

Published PCT application WO 2008/013416 A (AMOREPACIFIC CORPORATION) 31.01.2008 discloses a process for preparing a powder composition comprising nanoparticles of a sparingly water-soluble drug, which exhibits enhanced bioavailability and particle size stability of the drug, when dispersed in an aqueous medium.

<u>Solution to Problem</u>

In order to solve low bioavailability and low solubility of drug molecule, it is invented that olmesartan or pharmaceutically acceptable salts or esters thereof and solubility enhancing agent and preferably futher excipient or mixtures of excipients are intimately admixtured. Preferably olmesartan or pharmaceutically acceptable salts or esters thereof is mechanically treated and sieved with poloxamer in terms of admixturing. Poloxamer which is used in the range of 0.1 %- 15 %(w/w) of total pharmaceutical composition improve drug-membran interaction. This method does not require any supplemental investment or expenses.

**Description of embodiments**

In the present invention, inventors provide a pharmaceutical formulation and a production method to get a better bioavailability, solubility property by a reproducible, simple manufacturing method.

In the first aspect of this invention, olmesartan medoxomil is treated with poloxamer. Treatment can be numerous manners whatsoever. Accordingly, this invention delineates that preparation method of pharmaceutical composition of olmesartan or pharmaceutically acceptable salts and esters thereof or a preparation method of pharmaceutical combination of olmesartan or pharmaceutically acceptable salts and esters thereof characterized in that: a-) an intimate admixture of olmesartan or pharmaceutically acceptable salts and esters thereof and solubility enhancing agent and b-) said solubility enhancing agent is used in the range of 0.1 %-15% by total weight of pharmaceutical composition. An intimate admixture can be obtained, for example, by co-sieved, co-precipitation, co-milling, compression, granulation, or the like.

Poloxamers are water-soluble, nonionic, triblock copolymeric surfactants of poly(ethylene oxide) and poly(propylene oxide) .Among them, poloxamer 188 was approved by the Food and Drug Administration as a safe ingredient , it is reported in the National Formulary as a pharmaceutical ingredient.USP/NF specifies 5 different types of poloxamers with different chain lengths for a and b.

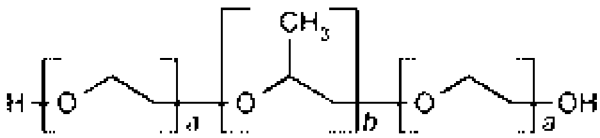

In solid preparations, poloxamer acts as wetting agent, plasticizer and solubility enhancing agent to improve the solubility and bioavailability of sparingly water soluble active drugs.

In this invention, olmesartan medoxomil and poloxamer particles are sized with a screen such as 315 microns but less than 710 microns. The drug is in intimate contact with the poloxamer in the particles. By this method, bioavailability of olmesartan is improved by increasing the surface area which enhances the bioavailability of olmesartan.

Olmesartan and poloxamer are sieved from a suitable milling equipment of preferably 315 microns. As an example, fitzmill enables comminution to a spesific particle range of active ingredient. By this process, active ingredient particles surface area is increased which helps dissolution and permeability of active ingredient. Furthermore, active ingredient particles are surrounded with poloxamer which improve the dissolution properties of drug as well.

In solid preparations, poloxamer acts as wetting agent, plasticizer and solubility enhancing agent to improve the solubility and bioavailability of sparingly water soluble active drugs.

According to this invention, poloxamer is used in an amount of 0.1%-15% (w /w) of total tablet weight is found to be optimum amount to improve the solubility, absorption and bioavailability of active ingredient.

In order to obtain better dissolution results, active ingredient and poloxamer mixtures are sized with different screens. Production by direct compression clearly showed that sifting low bio available active ingredient with poloxamer

from a preferably 315 microns sieve enhanced the solubility of drug.

Sized from preferably 315 microns screen, particles show better dissolution results compared to particles sized with 710 microns screen.

The dissolution profiles results obtained from different screens are summarized below.

**Table 1**

| Time (Minutes) | Dissolved % | | |
|---|---|---|---|
| | Sevikar® Daiichi Sankyo (Reference) | Olmesartan Containing Film Tablet wherein olmesartan and poloxamer sieved with 315 microns screen(Test) | Olmesartan Containing Film Tablet wherein olmesartan and poloxamer sieved with 710 microns screen(Test) |
| 10 | 58,9 | 56,8 | 49,6 |
| 15 | 65,6 | 65,9 | 59,6 |
| 20 | 70,9 | 72,3 | 66,0 |
| 30 | 76,8 | 78,5 | 73,1 |
| 45 | 81,8 | 82,6 | 78,7 |

Similarity factor $F_2$ value is found 89.1 for olmesartan containing film tablet wherein olmesartan medoxomil and poloxamer are sized by 315 microns screen when compared to the reference product. $F_2$ value is 60.5 for olmesartan containing film tablet wherein olmesartan medoxomil and poloxamer are sized by 710 microns screen when compared to the reference product. It shows that increased surface area of drug molecule which is treated with poloxamer improves the dissolution properties and bioavailability of the drug.

Thus an another aspect of this invention is that olmesartan medoxomil and poloxamer particles are sized with a screen less than 710 microns preferably 315 microns in terms of intimately admixturing.

According to this invention, the pharmaceutical compositions of olmesartan and active pharmaceutical ingredient or ingredients wherein formulations can be prepared by wet granulation, dry granulation, direct compression and the like. Said pharmaceutical composition may further comprise pharmaceutically acceptable excipients or excipients selected from the group consisting of diluents, binders, disintegrants, fillers, solubility enhancing agents, lubricants, glidants, mixtures thereof and the like.

The term 'filler' and the term 'diluent' are herein used interchangeably. Fillers fill out the size of a composition, making it practical to produce and convenient for the consumer to use. Suitable filler/diluent includes, but are not limited, to calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose lactose (e.g. spray-dried lactose, α-lactose, β-lactose, Tablettose®, various grades of Pharmatose®, Microtose® or Fast-Floc®), methylcellulose polymers such as, e.g., Methocel A®, Methocel A4C®, Methocel A 15C®, Metocel A4M®), hydroxyethylcellulose, hydroxypropylcellulose, L-hydroxypropylycellulose (low substituted), hydroxypropyl methylcellulose (HPMC) (e.g. Methocel E®, F and K, Metolose SH® of Shin-Etsu, grades of Methocel F® and Metolose 65 SH®, the 4,000, 15,000 and 100,000 cps grades of Methocel K®; and the 4,000, 15,000, 39,000 and 100,000 grades of Metolose 90 SH®), sodium carboxymethylcellulose, carboxymethylene, carboxymethylhydroxyethylcellulose and other cellulose derivatives, starches or modified starches ( including potato starch, wheat starch, corn starch, rice starch, pregelatinized maize starch), magnesium carbonate, magnesium oxide, maltitol, maltodextrins, maltose, sorbitol, starch, sucrose, sugar, and xylitol, erythritol.

A disintegrant is a substance which helps the composition break up once ingested. Disintegrants are, but not limited to, cross linked polyvinylpyrolidone (crospovidone, polyplyplasdone XL®, kollidon CL®); starches such as maize starch and dried sodium starch glycolate; gums such as maize starch and dried sodium starch glycolate; gums such as alginic acid, sodium alginate, guar gum; croscarmellose sodium; cellulose products such as microcrystalline cellulose and its salts, microfine cellulose, low-substituted hydroxypropylcellulose, mixtures thereof and the like.

Glidants improve the flowability of the composition. The composition may also comprise a glidant. Glidants are, but not limited to, colloidal silica, silicon dioxide, powdered cellulose, talc, tribasic calcium phosphate, mixtures thereof and the like.

Preferably, the solubility enhancing agent may be PEG-20-glyceryl stearate (Capmul® by Abitec), PEG-40 hydrogenated castor oil (Cremophor RH 40® by BASF), PEG 6 corn oil (Labrafil® by Gattefosse), lauryl macrogol - 32 glyceride (Gelucire 44/14® by Gattefosse), stearoyl macrogol glyceride (Gelucire 50/13® by Gattefosse), polyglyceryl - 10 mono dioleate (Caprol ® PEG 860 by Abitec), propylene glycol oleate (Lutrol OP® by BASF), propylene glycol

dioctanoate (Captex® by Abitec), propylene glycol caprylate/caprate (Labrafac® by Gattefosse), glyceryl monooleate (Peceol® by Gattefosse), glycerol monolinoleate (Maisine ® by Gattefosse), glycerol monostearate (Capmul® by Abitec), PEG- 20 sorbitan monolaurate (Tween 20® by ICI), PEG - 4 lauryl ether (Brij 30® by ICI), sucrose distearate (Sucroester 7® by Gattefosse), sucrose monopalmitate (Sucroester 15® by Gattefosse) ,polyoxyethylene-polyoxypropylene block copolymer (Lutrol® series BASF), polyethylene glycol 660 hydroxystearate, (Solutol® by BASF), sodium lauryl sulphate, sodium dodecyl sulphate, dioctyl suphosuccinate, L- hydroxypropyl cellulose, hydroxylethylcellulose, hydroxy propylcellulose, propylene glycol alginate, sodium taurocholate, sodium glycocholate, sodium deoxycholate, betains , polyethylene glycol (Carbowax® by DOW), d-α- tocopheryl polyethylene glycol 1000 succinate (Vitamin E TPGS® by Eastman), or mixtures thereof.

The presence of a lubricant is particularly preferred when the composition is a tablet as lubricants to improve the tabletting process. Lubricants prevent composition ingredients from clumping together and from sticking to the tablet punches or capsule filling machine and improve flowability of the composition mixture. Lubricants are, but not limited to sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, mixtures thereof and the like.

According to this invention, a pharmaceutical composition comprising olmesartan or pharmaceutically acceptable salt and esters, one or more active ingredient and poloxamer can also be prepared by direct compression.

In an embodiment of this invention, prepared by direct compression method, direct compression encompasses olmesartan or pharmaceutically acceptable salts and esters thereof and amlodipine or pharmaceutically acceptable salts thereof. Accordingly, combination comprises; olmesartan medoxomil is in the range of from about 5% to about 45%, amlodipine besylate is in the range of from about 2% to about 20%, poloxamer is in the range of from about 0.1 % to about 15%, disintegrant is in the range of from about 1% to about 15%, filler/diluent is in the range of from about 5% to about 80%, glidant is in the range of from about 0.1 % to about 10%, lubricant is in the range of from about 0.1 % to about 5%, coating agent is in the range of from about 0.1 % to about 10% by weight of total pharmaceutical composition.

Preferably, pharmaceutical composition comprising olmesartan medoxomil is 19.42%, amlodipine besylate is 6.73% , poloxamer is 2.00%, disintegrant is 4.85%, filler/diluent is 61.18 %, glidant is 2.18%, lubricant is 0.73%, coating agent is 2.92% % by the weight of total pharmaceutical composition.

**Table 2**

| **Total Pharmaceutical Composition (40/10)** | | |
|---|---|---|
| **Ingredients** | **mg** | **%** |
| Olmesartan Medoxomil | 40,00 | 19,42 |
| Amlodipine Besylate | 13,86 | 6,73 |
| Poloxamer | 4,12 | 2,00 |
| Filler/Diluent | 126,02 | 61,18 |
| Disintegrant | 10,00 | 4,85 |
| Glidant | 4,50 | 2,18 |
| Lubricant | 1,50 | 0,73 |
| Coating Agent | 6,00 | 2,92 |
| **Total** | **206,00** | **100,00** |

On the other hand this invention includes a preparation method for direct compression of olmesartan or pharmaceutically acceptable salts and esters and amlodipine or pharmaceutically acceptable salts. This method comprising steps of a. Olmesartan medoxomil and poloxamer are sieved by using a screen of 315 microns and installed into container and mixed. b. A portion of filler/diluent is added to powder prepared at step (a) and mixed. c. Amlodipine besylate, a portion of filler are sieved by using suitable equipment and mixed. d. A portion of filler/diluent and disintegrant are added to the mixture prepared at step (b) e. The mixture prepared at step (c) and (d) are mixed f. Pre-sieved lubricant and pre-sieved glidant are installed into container onto powder mixture prepared at step e and mixed. g. The final mixture is compressed h. A Coating agent or mixtures of coating agents are added into solvent and stirred. i. Core tablets are installed into a coating pan and coated with the coating suspension prepared at step h.

According to the present invention, pharmaceutical compositions are preferably prepared by a wet granulation process.

In an embodiment of this invention, the process for preparing solid oral dosage forms comprising combination of olmesartan or pharmaceutically acceptable salts and esters thereof and amlodipine or pharmaceutically acceptable salts wherein composition has intra-granulation step and intra-granular ingredient(s) and extra-granulation step and extra-granular ingredient(s) .It comprises steps of:

**a. Intragranulation**

1. Olmesartan medoxomil and poloxamer are mixed and sieved by using a screen of 315 microns.
2. Binder and solvent are mixed until binder is dissolved.
3. Mixture prepared at step (1), amlodipine besylate, filler and a portion of disintegrant are mixed in the granulator.
4. The granulation solution prepared at step 2 is added onto the mixture in the granulator to form granules.
5. The blend is mixed and transferred to a fluidized bed dryer until proper moisture then sizing the dried granules by using suitable milling equipment.

**b.Extragranulation**

1. Granules of internal phase are transferred to the mixer, a portion of disintegrant and additional filler are added to the mixture.
2. Pre-sieved glidant and lubricant are added to the mixture and mixed.
3. The final blend is compressed on a tabletting machine using appropriate punches.
4. Coating agent or mixtures of coating agents are added into purified water and stirred.
5. Core tablets are installed into a coating pan and coated with the coating suspension prepared at step (4).
In a further embodiment, solvents used for granulation process may be selected from water, isopropyl alcohol, acetone, ethanol, methylene chloride mixtures thereof and the like.
In another aspect of this invention, intragranulation comprising olmesartan medoxomil is from about 5% to about 45%, amlodipine besylate is from about 2% to about 20%, poloxamer is from about 0.1 % to about 15%, filler /diluent is from about 5% to about 80%, disintegrant is from about 1 % to about 15%, and binder is from about 0.1 % to about 5% by weight of intragranular phase's ingredients.
Preferably, intragranulation comprising olmesartan medoxomil is 31.24%, amlodipine besylate is 10.82%, poloxamer is 3.28%, filler/diluent is 46.85%, disintegrant is 4.69%, binder is 3.12% by total weight of intragranulation phase's ingredients.

**Table 3**

| **Intragranulation (40/10-20/5)** | | |
|---|---|---|
| **Ingredients** | **mg** | **%** |
| Olmesartan Medoxomil | 40,00 | 31,24 |
| Amlodipine Besylate | 13,86 | 10,82 |
| Poloxamer | 4,20 | 3,28 |
| Binder | 4,00 | 3,12 |
| Filler | 60,00 | 46,85 |
| Disintegrant | 6,00 | 4,69 |
| **Total** | 128,06 | 100,00 |

Extragranulation comprising disintegrant is from about 1% to about 15%, filler/diluent is from about 5% to about 80%, glidant is from about 0.1 % to about 10%, lubricant is from about 0.1 % to about 5%, and coating agent is from about 0.1 % to about 10 by total weight of extragranular phase's excipients.
Preferably, extragranulation comprising disintegrant is 7.70%, filler is 76.98%, glidant is 5.70%, lubricant is 1.92 %, coating agent is 7.70% by total weight of extragranulation step's excipients.

Table 4

| Extragranulation(40/10-20/5) | | |
|---|---|---|
| **Ingredients** | **mg** | **%** |
| Disintegrant | 6,00 | 7,70 |
| Filler/Diluent | 60,00 | 76,98 |
| Glidant | 4,44 | 5,70 |
| Lubricant | 1,50 | 1,92 |
| Coating Agent | 6,00 | 7,70 |
| **Total** | 77,94 | 100,00 |

In an embodiment of this invention, olmesartan medoxomil is in the range of from about %5 to about 45%, amlodipine besylate is in the range of from about %2 to about 20%, poloxamer is in the range of from about 0.1 % to about 15%, binder is in the range of from about %0.1 to about 5%, disintegrant is in the range of from about 1% to about 15%, filler/diluent is in the range of from about 5% to about 80%, glidant is in the range of from about 0.1 % to about 10%, lubricant is in the range of from about 0.1 % to about 5%, coating agent is in the range of from about 0.1 % to about 10% by weight of total pharmaceutical composition.

Preferably, pharmaceutical composition comprising olmesartan medoxomil thereof is 19.42%, amlodipine besylate is 6.73%, poloxamer is 2.04%, binder is 1.94%, disintegrant is 5.82%, filler/diluent is 58.26 %, glidant is 2.16%, lubricant is 0.73%, coating agent is 2.91 % by the weight of total pharmaceutical composition.

Table 5

| Total Pharmaceutical Composition (40/10-20/5) | | |
|---|---|---|
| **Ingredients** | **mg** | **%** |
| Olmesartan Medoxomil | 40,00 | 19,42 |
| Amlodipine Besylate | 13,86 | 6,73 |
| Poloxamer | 4,20 | 2,04 |
| Binder | 4,00 | 1,94 |
| Disintegrant | 12,00 | 5,82 |
| Filler/Diluent | 120,00 | 58,26 |
| Glidant | 4,44 | 2,16 |
| Lubricant | 1,50 | 0,73 |
| Coating Agent | 6,00 | 2,91 |
| **Total** | **206,00** | **100,00** |

In another aspect of this invention, in 40 mg Olmesartan Medoxomil /5 mg amlodipine besylate formulations, intragranulation comprising olmesartan medoxomil is from about 5% to about 45%, amlodipine besylate is from about 2% to about 20%, poloxamer is from about 0.1 % to about 15%, filler/diluent is from about 5% to about 80%, disintegrant is from about 1% to about 15% and binder is from about 0.1 % to about 5% by weight of intragranular phase's ingredients.

Preferably, in 40 mg olmesartan medoxomil /5 mg amlodipine besylate formulations, intragranulation comprising olmesartan medoxomil is 33.02%, amlodipine besylate is 5.72%, poloxamer is 3.47%, filler/diluent is 49.53%, disintegrant is 4.95%, binder is 3.30% by total weight of intragranulation phase's ingredients.

Table 6

| Intragranulation (40/5) | | |
|---|---|---|
| **Ingredients** | **mg** | **%** |
| Olmesartan Medoxomil | 40,00 | 33,02 |

(continued)

| Intragranulation (40/5) | | |
|---|---|---|
| **Ingredients** | **mg** | **%** |
| Amlodipine Besylate | 6,93 | 5,72 |
| Poloxamer | 4,20 | 3,47 |
| Binder | 4,00 | 3,30 |
| Filler/Diluent | 60,00 | 49,53 |
| Disintegrant | 6,00 | 4,95 |
| **Total** | **121,13** | **100,00** |

In 40 mg Olmesartan medoxomil /5 mg amlodipine besylate formulations, extragranulation comprising disintegrant is from about 1% to about 15%, filler/diluent is from about 5% to about 80%, glidant is from about 0.1 % to about 10 %, lubricant is from about 0.1 % to about 5%, and coating agent is from about 0.1 % to about 10 by total weight of extragranular phase's excipients.

In 40 mg Olmesartan Medoxomil /5 mg amlodipine besylate formulations, extragranulation preferably comprising disintegrant is 7.07%, filler is 78.86%, glidant is 5.23%, lubricant is 1.77 %, coating agent is 7.07% by total weight of extragranulation step's excipients.

**Table 7**

| Extragranulation(40/5) | | |
|---|---|---|
| **Ingredients** | **mg** | **%** |
| Disintegrant | 6,00 | 7,07 |
| Filler/Diluent | 66,93 | 78,86 |
| Glidant | 4,44 | 5,23 |
| Lubricant | 1,50 | 1,77 |
| Coating Agent | 6,00 | 7,07 |
| **Total** | **84,87** | **100,00** |

In 40 mg olmesartan medoxomil /5 mg amlodipine besylate formulations, olmesartan medoxomil is in the range of from about %5 to about 45%, amlodipine besylate is in the range of from about %2 to about 20%, poloxamer is in the range of from about 0.1 % to about 15%, binder is in the range of from about % 0.1 to about 5%, disintegrant is in the range of from about 1% to about 15%, filler/diluent is in the range of from about 5% to about 80%, glidant is in the range of from about 0.1 % to about 10%, lubricant is in the range of from about 0.1 % to about 5%, coating agent is in the range of from about 0.1 % to about 10% by weight of total pharmaceutical composition.

In 40 mg olmesartan medoxomil /5 mg amlodipine besylate formulations, pharmaceutical composition preferably comprising olmesartan medoxomilthereof is 19.42%, amlodipine besylate is 3.36% ,poloxamer is 2.04%, binder is 1.94%, disintegrant is 5.82%, filler/diluent is 58.26 %, glidant is 2.16%, lubricant is 0.73%, coating agent is 2.91 % by the weight of total pharmaceutical composition.

**Table 8**

| Total Pharmaceutical Composition (40/5) | | |
|---|---|---|
| **Ingredients** | **mg** | **%** |
| Olmesartan Medoxomil | 40,00 | 19,42 |
| Amlodipine Besylate | 6,93 | 3,36 |
| Poloxamer | 4,20 | 2,04 |
| Binder | 4,00 | 1,94 |

(continued)

| Total Pharmaceutical Composition (40/5) | | |
|---|---|---|
| **Ingredients** | **mg** | **%** |
| Disintegrant | 12,00 | 5,82 |
| Filler/Diluent | 126,93 | 58,26 |
| Glidant | 4,44 | 2,16 |
| Lubricant | 1,50 | 0,73 |
| Coating Agent | 6,00 | 2,91 |
| **Total** | **206,00** | **100,00** |

In another aspect of this invention, in 20 mg Olmesartan Medoxomil/10 mg amlodipine besylate formulations, intragranulation comprising olmesartan medoxomil is from about 5% to about 45%, amlodipine besylate is from about 2% to about 20%, poloxamer is from about 0.1 % to about 15%, filler/diluent is from about 5% to about 80% disintegrant is from about 1 % to about 15% and binder is from about 0.1 % to about 5% by weight of intragranular phase's ingredients.

In 20 mg Olmesartan Medoxomil/10 mg amlodipine besylate formulations ,preferably, intragranulation comprising olmesartan medoxomil is 16.94%, amlodipine besylate is 11.74%, poloxamer is 1.78%, filler/diluent is 61.07%, disintegrant is 5.08%, binder is 3.39% by total weight of intragranulation phase's ingredients.

**Table 9**

| Intragranulation (20/10) | | |
|---|---|---|
| **Ingredients** | **mg** | **%** |
| Olmesartan Medoxomil | 20,00 | 16,94 |
| Amlodipine Besylate | 13,86 | 11,74 |
| Poloxamer | 2,10 | 1,78 |
| Binder | 4,00 | 3,39 |
| Filler/Diluent | 72,10 | 61,07 |
| Disintegrant | 6,00 | 5,08 |
| **Total** | **118,06** | **100,00** |

In an another aspect of this invention, in 20 mg olmesartan medoxomil/10 mg amlodipine besylate formulations, extragranulation comprising disintegrant is from about 1% to about 15%, filler/diluent is from about 5% to about 80%, glidant is from about 0.1 % to about 10%, lubricant is from about 0.1 % to about 5%, and coating agent is from about 0.1% to about 10% by total weight of extragranular phase's excipients.

In 20 mg olmesartan medoxomil/10 mg amlodipine besylate formulations, preferably, extragranulation comprising disintegrant is 6.82%, filler/diluent is 79.60%, glidant is 5.05%, lubricant is 1.71 %, coating agent is 6.82% by total weight of extragranulation step's excipients.

**Table 10**

| Extragranulation(20/10) | | |
|---|---|---|
| **Ingredients** | **mg** | **%** |
| Disintegrant | 6,00 | 6,82 |
| Filler/Diluent | 70,00 | 79,60 |
| Glidant | 4,44 | 5,05 |
| Lubricant | 1,50 | 1,71 |
| Coating Agent | 6,00 | 6,82 |
| **Total** | **87,94** | **100,00** |

In an embodiment of this invention, in aspect of 20 mg olmesartan medoxomil/10 mg amlodipine besylate formulations, olmesartan medoxomil is in the range of from about %5 to about 45%, amlodipine besylate is in the range of from about %2 to about 20%, poloxamer is in the range of from about 0.1 % to about 15%, binder is in the range of from about %0.1 to about 5%, disintegrant is in the range of from about 1% to about 15%, filler/diluent is in the range of from about 5% to about 80%, glidant is in the range of from about 0.1 % to about 10%, lubricant is in the range of from about 0.1 % to about 5%, coating agent is in the range of from about 0.1 % to about 10% by weight of total pharmaceutical composition.

In 20 mg Olmesartan Medoxomil /10 mg amlodipine besylate formulations,preferably, pharmaceutical composition comprising olmesartan medoxomil is 9.71 %, amlodipine besylate is 6.73% , poloxamer is 1.02%, binder is 1.94%, disintegrant is 5.82%, filler/diluent is 68.98 %, glidant is 2.16%, lubricant is 0.73%, coating agent is 2.91% by the weight of total pharmaceutical composition.

**Table 11**

| Total Pharmaceutical Composition (20/10) | | |
|---|---|---|
| **Ingredients** | **mg** | **%** |
| Olmesartan Medoxomil | 20,00 | 9,71 |
| Amlodipine Besylate | 13,86 | 6,73 |
| Poloxamer | 2,10 | 1,02 |
| Binder | 4,00 | 1,94 |
| Disintegrant | 12,00 | 5,82 |
| Filler/Diluent | 142,10 | 68,98 |
| Glidant | 4,44 | 2,16 |
| Lubricant | 1,50 | 0,73 |
| Coating Agent | 6,00 | 2,91 |
| **Total** | **206,00** | **100,00** |

A suitable dissolution method is used to reach dissolution profiles. In this invention USP Type-2 apparatus is preferred and used. According to the method which was developed during drug development process, the tablets prepared in accordance with the present invention should exhibit the following dissolution profile when tested in a USP Type 2 apparatus, at 50 rpm, and 37° C,900 ml and in pH=6,8 phosphate buffer medium.

To determine the similarity between the dissolution profiles of the test and reference product a simple model independent approach, that is $f_2$ (similarity factor), was carried out. As per US FDA, $f_2$ values should lie between 50 and 100 for developing two similar dissolution profiles.

The similarity factor $f_2$ is a measurement of the similarity through a point by point comparison as shown in equation 1.

$$f_2 = 50 * \log \frac{100}{\sqrt{1 + \frac{1}{n}\sum_{t=1}^{n}(R_t - T_t)^2}}$$

n: is the number of sampling time points

Rt : is the amount drug released from a reference batch at time t

Tt : is the amount drug released from a test batch at time t.

Generally, f2 values greater than 50 ensure sameness of the performance of the reference product and test product.

The test product and the reference product's results show that the $f_2$ is in the limits of 50-100 as established by the US FDA for claiming similarity between the dissolution profiles of the test and reference product.

Olmesartan medoxomil test tablet is released in pH=6,8 phosphate buffer environment under conditions of 900

ml of a dissolution medium at 37 C, USP method-II (pedal) 50 rpm wherein tablet exhibits a dissolution profile.(Table 12, Figure 1).$F_2$ value is 98.7.

The results obtained by wet granulation method are summarized below table.

**Table 12**

| Time (Minutes ) | Dissolved % | |
|---|---|---|
| | Sevikar® Daiichi Sankyo (Reference) | Olmesartan Film Tablet (Test) |
| 10 | 60,6 | 60,6 |
| 15 | 68,1 | 68,5 |
| 20 | 72,8 | 72,7 |
| 30 | 78,8 | 78,2 |
| 45 | 82,3 | 82,1 |

## Claims

1. A preparation method of pharmaceutical composition of olmesartan or pharmaceutically acceptable salts and esters thereof or a preparation method of pharmaceutical combination of olmesartan or pharmaceutically acceptable salts thereof with an active pharmaceutical ingredient or mixtures of active pharmaceutical ingredients **characterized in that**: a-) an intimate admixture of olmesartan or pharmaceutically acceptable salts thereof and solubility enhancing agent and preferably further excipient or mixtures of excipients and b-) said solubility enhancing agent is used in the range of 0.1 %-15% by total weight of pharmaceutical composition.

2. According to claim 1, pharmaceutical composition is prepared by direct compression and wet granulation methods.

3. According to claim 2, pharmaceutical composition prepared by direct compression method wherein pharmaceutical composition comprising: olmesartan medoxomil is in the range of from 5% to 45%, amlodipine besylate is in the range of from 2% to 20%, poloxamer is in the range of from 0.1 % to 15%, disintegrant is in the range of from 1% to 15%, filler/diluent is in the range of from 5% to 80%, glidant is in the range of from 0.1 % to 10%, lubricant is in the range of from 0.1 % to 5%, coating agent is in the range of from 0.1 % to 10% by weight of total pharmaceutical composition.

4. According to claim 3, pharmaceutical composition preferably comprising: pharmaceutical composition comprising olmesartan medoxomil is 19.42%, amlodipine besylate is 6.73% ,poloxamer is 2.00%, disintegrant is 4.85%, filler/diluent is 61.18 %, glidant is 2.18%, lubricant is 0.73%, coating agent is 2.92% by the weight of total pharmaceutical composition.

5. According to claim 2, pharmaceutical composition prepared by wet granulation method wherein pharmaceutical composition comprising : olmesartan medoxomil is in the range of from %5 to 45%, amlodipine besylate is in the range of from %2 to 20%,poloxamer is in the range of from 0.1 % to 15%, binder is in the range of from %0.1 to 5%, disintegrant is in the range of from 1% to 15%, filler/diluent is in the range of from 5% to 80%, glidant is in the range of from 0.1 % to 10%, lubricant is in the range of from 0.1 % to 5%, coating agent is in the range of from 0.1 % to 10% by weight of total pharmaceutical composition.

6. According to claim 5, **a-)** in 40 mg Olmesartan Medoxomil/10 mg amlodipine besylate and in 20 mg Olmesartan Medoxomil /5 mg amlodipine besylate formulations pharmaceutical composition preferably comprising: olmesartan medoxomil is 19.42%, amlodipine besylate is 6.73%, poloxamer is 2.04%, binder is 1.94%, disintegrant is 5.82%, filler/diluent is 58.26 %, glidant is 2.16%, lubricant is 0.73%, coating agent is 2.91 % by the weight of total pharmaceutical composition, **b-)** In 40 mg Olmesartan Medoxomil/5mg amlodipine besylate formulations pharmaceutical composition preferably comprising : olmesartan medoxomil is 19.42%, amlodipine besylate is 3.36% ,poloxamer is 2.04%, binder is 1.94%, disintegrant is 5.82%, filler/diluent is 58.26 %, glidant is 2.16%, lubricant is 0.73%, coating agent is 2.91 % by the weight of total pharmaceutical composition,**c-)** In 20 mg Olmesartan Medoxomil/10 mg amlodipine besylate formulations pharmaceutical composition preferably comprising olmesartan medoxomil is 9.71 %, amlodipine besylate is 6.73% , poloxamer is 1.02%, binder is 1.94%, disintegrant is 5.82%, filler/diluent is 68.98

%, glidant is 2.16%, lubricant is 0.73%, coating agent is 2.91 % by the weight of total pharmaceutical composition.

**7.** According to claim 5, wet granulation method comprising intragranulation and extragranulation steps.

**8.** According to claim 7, intragranulation step comprising: olmesartan medoxomil is in the range of from 5% to 45%, amlodipine besylate is in the range of from 2% to 20%, poloxamer is in the range of from 0.1 % to 15%, filler/diluent is in the range of from 5% to 80% disintegrant is in the range of from 1% to 15% and binder is in the range of from 0.1 % to 5% by weight of intragranular phase's ingredients.

**9.** According to claim 8, a-) in 40 mg Olmesartan Medoxomil/10 mg amlodipine besylate and in 20 mg Olmesartan Medoxomil /5 mg amlodipine besylate formulations, intragranulation preferably comprising: olmesartan medoxomil is 31.24%, amlodipine besylate is 10.82%, poloxamer is 3.28%, filler/diluent is 46.85%, disintegrant is 4.69%, binder is 3.12% by total weight of intragranulation phase's ingredients, b-) In 40 mg Olmesartan Medoxomil /5 mg amlodipine besylate formulations, intragranulation preferably comprising olmesartan medoxomil is 33.02%, amlodipine besylate is 5.72%, poloxamer is 3.47%, filler/diluent is 49.53%, disintegrant is 4.95%, binder is 3.30% by total weight of intragranulation phase's ingredients, c-)In 20 mg Olmesartan Medoxomil /10 mg amlodipine besylate formulations , intragranulation preferably comprising olmesartan medoxomil is 16.94%, amlodipine besylate is 11.74%, poloxamer is 1.78%, filler/diluent is 61.07%, disintegrant is 5.08%, binder is 3.39% by total weight of intragranulation phase's ingredients.

**10.** According to claim 7, extragranulation step comprising: disintegrant is in the range of from 1% to 15%, filler/diluent is in the range of from 5% to 80%, glidant is in the range of from 0.1 % to 10%, lubricant is in the range of from 0.1 % to 5%, and coating agent is in the range of from 0.1 % to10% by total weight of extragranular phase's excipients.

**11.** According to claim 10, α-)in 40 mg olmesartan medoxomil/10 mg amlodipine besylate and 20 mg Olmesartan Medoxomil /5 mg amlodipine besylate formulations,extragranulation preferably comprising: disintegrant is 7.70%, filler/diluent is 76.98%, glidant is 5.70%, lubricant is 1.92 %, coating agent is 7.70% by total weight of extragranulation step's excipients b-)In 40 mg Olmesartan Medoxomil /5 mg amlodipine besylate formulations, extragranulation preferably comprising disintegrant is 7.07%, filler is 78.86%, glidant is 5.23%, lubricant is 1.77 %, coating agent is 7.07% by total weight of extragranulation step's excipients,c-)In 20 mg Olmesartan Medoxomil/10 mg amlodipine besylate formulations, extragranulation preferably comprising: disintegrant is 6.82%, filler/diluent is 79.60%, glidant is 5.05%, lubricant is 1.71 %, coating agent is 6.82% by total weight of extragranulation step's excipients.

**12.** According to claim 2, a wet granulation process for preparing pharmaceutical composition comprises the following steps: a. Olmesartan medoxomil and poloxamer are mixed and sieved by using a screen of 315 microns. b. Binder and solvent are mixed until binder is dissolved. c. Mixture prepared at step (a), amlodipine besylate, filler and a portion of disintegrant are mixed in the granulator. d. The granulation solution prepared at step (b) is sprayed onto the mixture in the granulator to form granules. e. The blend is mixed and transferred to a fluidized bed dryer until proper moisture then sizing the dried granules by using suitable milling equipment. f. Granules of internal phase are transferred to the mixer, a portion of disintegrant and additional filler are added to the mixture.g. Pre-sieved glidant and lubricant are added to the mixture and mixed. h. The final blend is compressed on a tabletting machine using appropriate punches. i. Coating agent or mixtures of coating agents are added into purified water and stirred. j. Core tablets are installed into a coating pan and coated with the coating suspension prepared at step (i).

**13.** According to claim 2, a direct compression process for preparing pharmaceutical composition comprises the following steps: a. Olmesartan medoxomil and poloxamer are sieved by using a screen of 315 microns and installed into container and mixed. b. A portion of filler/diluent is added to powder prepared at step (a) and mixed. c. Amlodipine besylate, a portion of filler are sieved by using suitable equipment and mixed. d. A portion of filler/diluent and disintegrant are added to the mixture prepared at step (b) e. The mixture prepared at step (c) and (d) are mixed f. Pre-sieved glidant and pre-sieved lubricant are installed into container onto powder mixture prepared at step e and mixed. g. The final mixture is compressed h. A Coating agent or mixtures of coating agents are added into solvent and stirred. i. Core tablets are installed into a coating pan and coated with the coating suspension prepared at step h.

**14.** According to claim 1, solubility enhancing agent is poloxamer.

**15.** According to preceding claims olmesartan medoxomil and poloxamer particles are sized with a screen less than 710 microns in terms of intimately admixturing.

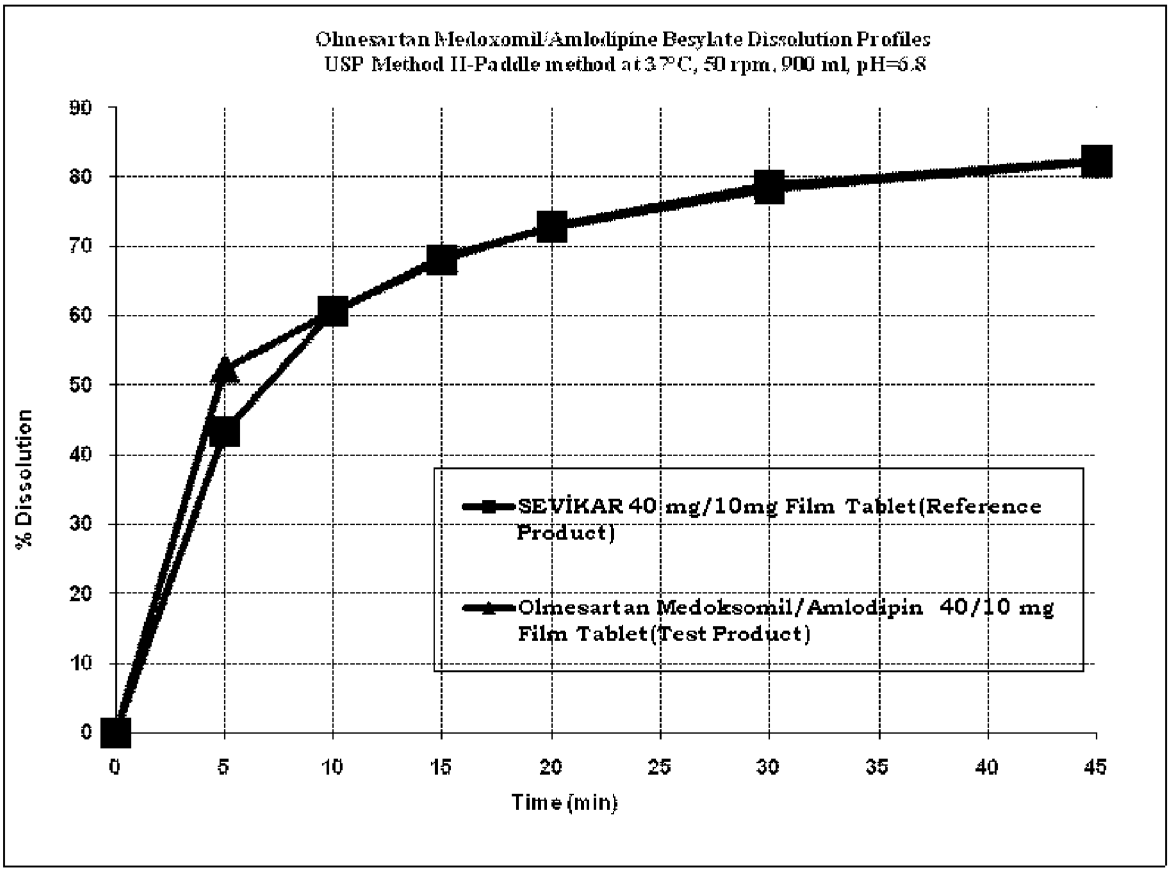
Olmesartan Medoxomil/Amlodipine Besylate Dissolution Profiles
USP Method II-Paddle method at 37°C, 50 rpm, 900 ml, pH=6.8
% Dissolution
Time (min)
0
10
20
30
40
50
60
70
80
90
0
5
10
15
20
25
30
35
40
45
SEVİKAR 40 mg/10mg Film Tablet(Reference Product)
Olmesartan Medoksomil/Amlodipin 40/10 mg Film Tablet(Test Product)

Fig. 1

Europäisches Patentamt
European Patent Office
Office européen des brevets

# EUROPEAN SEARCH REPORT

Application Number
EP 10 17 2244

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/001065 A2 (SANKYO CO [JP]; HAMAURA TAKESHI [JP]; KANNO MITSURU [JP]) 4 January 2007 (2007-01-04)<br>* page 1, paragraph 1 *<br>* page 2, line 1 - page 4, line 30 *<br>* page 9, line 30 - page 15, line 5 *<br>* page 16, line 11 - line 16; examples 1, reference 1, 2 , reference 2 *<br>* claims 1, 4, 7-10, 15-19, 26 *<br>----- | 1-15 | INV.<br>A61K47/10<br>A61K9/20<br>A61K9/16<br>A61K9/14<br>A61K9/50<br>A61K31/4178<br>A61K45/06 |
| X,D | EP 2 033 643 A1 (DAIICHI SANKYO CO LTD [JP]) 11 March 2009 (2009-03-11)<br>* paragraph [0004] - paragraph [0007] *<br>* paragraph [0013] - paragraph [0032] *<br>* claims 1, 6-8 *<br>----- | 1-15 | |
| X | WO 2008/032107 A1 (DAIICHI SANKYO CO LTD [JP]; FAIRBAIRN ANGUS CHRISHOLM [GB]; BAUER WOLF) 20 March 2008 (2008-03-20)<br>* page 4, line 4 - page 5, line 10 *<br>* page 13, line 5 - page 15, line 10 *<br>* page 16, line 10 - page 17, line 4 *<br>* example 1 *<br>* claims 1, 30, 31, 35, 36 *<br>----- | 1-15 | TECHNICAL FIELDS SEARCHED (IPC)<br>A61K |
| X | US 2010/119607 A1 (PALEPU NAGESH R [US] ET AL) 13 May 2010 (2010-05-13)<br>* paragraph [0015] - paragraph [0018] *<br>* paragraph [0031] - paragraph [0057] *<br>* tables 10, 12, 15 *<br>* claims 48-57, 62, 63 *<br>-----<br>-/-- | 1-15 | |

The present search report has been drawn up for all claims

| Place of search | Date of completion of the search | Examiner |
|---|---|---|
| The Hague | 20 December 2010 | González Ferreiro, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 10 17 2244

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/084040 A1 (RUBICON RES PRIVATE LTD [IN]; PILGAONKAR PRATIBHA SUDHIR [IN]; RUSTOMJ) 9 July 2009 (2009-07-09)<br>* page 7, line 16 - line 27 *<br>* page 13, line 29 - page 14, line 25 *<br>* page 16, line 13 - page 7, line 24 *<br>* page 19, line 15 - page 21, line 5; claims 1, 2, 7-9, 12-14, 17 *<br>----- | 1-15 | |
| A | WO 2009/110010 A2 (SUN PHARMACEUTICAL IND LTD [IN]; DHAVSE VAISHALI VIJAY [IN]; DHARMADHI) 11 September 2009 (2009-09-11)<br>* page 3, line 22 - line 27 *<br>* page 5, line 22 - line 24 *<br>* page 7, line 33 - line 19 *<br>* claims 1, 5, 7, 9, 11 *<br>----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 20 December 2010 | González Ferreiro, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO.

EP 10 17 2244

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2010

| Patent document cited in search report | Publication date | Patent family member(s) | Publication date |
|---|---|---|---|
| WO 2007001065 A2 | 04-01-2007 | JP 2008543727 T | 04-12-2008 |
| EP 2033643 A1 | 11-03-2009 | CA 2656181 A1 | 03-01-2008 |
| | | CN 101478966 A | 08-07-2009 |
| | | WO 2008001734 A1 | 03-01-2008 |
| | | KR 20090021184 A | 27-02-2009 |
| | | US 2010237530 A1 | 23-09-2010 |
| WO 2008032107 A1 | 20-03-2008 | AU 2007297333 A1 | 20-03-2008 |
| | | DE 212007000063 U1 | 14-05-2009 |
| | | GB 2454620 A | 13-05-2009 |
| | | SE 0900332 A | 12-06-2009 |
| | | TR 200901984 T1 | 21-08-2009 |
| | | US 2009175942 A1 | 09-07-2009 |
| | | ZA 200810616 A | 26-08-2009 |
| US 2010119607 A1 | 13-05-2010 | NONE | |
| WO 2009084040 A1 | 09-07-2009 | NONE | |
| WO 2009110010 A2 | 11-09-2009 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0503785 B
- EP 244944 B
- WO 2008149338 A
- EP 2033643 A
- WO 2007001066 A
- WO 2008013416 PCT